# EUROPEAN PATENT APPLICATION

(11) **EP 4 775 664 A1**
(43) Date of publication of application: **15.07.2026**
(21) Application number: 25217764.7
(22) Date of filing: 21.11.2025
(51) Int. Cl.: C12N 1/205, C12P 19/04, A61K 31/715, A61P 3/04, C08B 37/00

(54) **STRAIN OF DUGANELLA SP., EXOPOLYSACCHARIDE PRODUCED THEREBY, AND USE**

(30) Priority: 14.01.2025 CN 202510057031
(71) Applicant: Nanjing Southern Element Biotechnology Co., Ltd., Nanjing City, Jiangsu 210031 (CN)
(72) Inventor: ZHANG, Jianfa, Nanjing City, 210031 (CN); XU, Xi, Nanjing City, 210031 (CN); XU, Xiaodong, Nanjing City, 210031 (CN)
(74) Representative: Canzler & Bergmeier Patentanwälte Partnerschaft mbB

(57) **Abstract**

A strain of *Duganella* sp., a polysaccharide produced thereby, and use are provided. The *Duganella* sp. G3 of the present disclosure has the deposit number of CCTCC NO: M 2024137. The exopolysaccharide TDG3 produced by the strain acts as a TLR4/DC-SIGN dual receptor agonist and significantly reduces body weight. The exopolysaccharide TDG3 of the present disclosure may significantly reduce appetite in mice, decrease food take, lower body weight, and reduce fat mass. It may also alleviate or prevent obesity-induced hepatic steatosis. It is safe and non-toxic, suitable for the preparation of lipid-lowering and weight-loss drugs, and has good clinical application value and broad application prospects.

## Description

### TECHNICAL FIELD

The present disclosure belongs to the technical field of microbial exopolysaccharides, and relates to a strain of *Duganella* sp., an exopolysaccharide produced thereby, and use.

### BACKGROUND

Obesity affects approximately 19% of women and 14% of men worldwide and is associated with increased morbidity. Anti-obesity medications can alter biological processes affecting appetite and significantly improve outcomes, such as type 2 diabetes, hypertension, and dyslipidemia. Bariatric surgery is the most effective treatment for weight loss in patients with morbid obesity, but we need less aggressive treatments. An ideal weight-loss drug should have the following characteristics: effectiveness and safety. In particular, it should effectively reduce hunger and suppress the desire to eat, thereby significantly alleviating obesity symptoms and achieving a significant reduction in body weight and fat mass. In terms of safety, while exerting the weight-loss efficacy, the drug must ensure that adverse side effects such as organ damage and cardiovascular disease are not caused, thereby avoiding any potential threats to health. It should focus on reducing those excessive, abnormal fat tissues that are detrimental to health, while meticulously protecting muscles and bones to ensure a healthy and comprehensive weight-loss process. Furthermore, an ideal weight-loss drug should also have the convenient usage characteristics, i.e., low frequency of administration and long-lasting pharmacological effect. It is preferable that it can align with the rhythm of people's daily lives, for example, requiring administration only once per week or even at longer intervals, thereby ensuring that the weight-loss process does not interfere with the quality of life and well-being of individuals due to frequent medication.

Weight-loss drugs currently on the market and in the research field can be classified into various types on the basis of their different mechanisms of action. Taking orlistat as an example, this drug achieves a weight-loss effect primarily by blocking the absorption of dietary fats and enhancing patient acceptance of low-fat diets and snacks. However, the use of orlistat may be accompanied by a series of mild to moderate gastrointestinal discomforts, such as symptoms of oily stools, diarrhea, abdominal pain, and even blood in the stool. More seriously, a few case reports have indicated that it may cause serious liver adverse effects, including cholelithiasis, cholestatic hepatitis, and even subacute liver failure. Another class of centrally acting drugs, such as phentermine-topiramate and naltrexone-bupropion, achieve weight loss by regulating the appetite center in the brain. Approximately 20% of patients taking these drugs experience constipation, while the incidence of other adverse effects (such as paresthesia and nausea) varies depending on the drug type. Drugs based on GLP-1 receptor agonists, such as liraglutide and semaglutide, by mimicking the action of enteropancreatic hormones, not only alter central appetite regulation mechanism, but also provide a variety of cardiometabolic weight-loss benefits. However, these drugs are also associated with a series of adverse effects, including nausea (incidence 28%-44%), diarrhea (21%-30%), and constipation (11%-24%).

In summary, although a variety of weight-loss drugs currently available have demonstrated good weight-loss efficacy in clinical practice, they are all accompanied by adverse effects of varying degrees. In addition, not all patients achieve sufficient weight loss in response to existing drugs. Therefore, there is an urgent need to develop new weight-loss drugs to provide new ideas for the research and development of new weight-loss drugs.

### SUMMARY

The present disclosure provides a strain of *Duganella* sp., an exopolysaccharide produced thereby, and use.

The *Duganella* sp. according to the present disclosure is a strain of *Duganella* sp. G3, which was deposited in the China Center for Type Culture Collection (CCTCC) on January 18, 2024, with the deposit number of CCTCC NO: M 2024137 and the deposit address: Wuhan University, Wuhan, China.

A method for culturing the above *Duganella* sp. G3 specifically includes: inoculating the *Duganella* sp. G3 into a fermentation medium for culture, where the culture is performed at a pH of 5 to 9, and a temperature of 26°C to 32°C.

The fermentation medium according to the present disclosure is a medium conventionally used for *Duganella* sp., for example, a fermentation medium with the following formulation may be used: carbon source 20 to 50 g/L, nitrogen source 1 to 4 g/L, NaH₂PO₄ 0.5 to 2 g/L, CaCl₂ 0.02 to 0.1 g/L, MgSO₄·7H₂O 0.1 to 0.5 g/L, FeSO₄·7H₂O 0.01 to 0.06 g/L, MnSO₄·H₂O 0.005 to 0.01 g/L, and ZnCl₂ 0.01 to 0.02 g/L. The carbon source in the fermentation medium according to the present disclosure is a carbon source conventionally used for *Duganella* sp., for example, one or more selected from the group consisting of glucose, sucrose, starch.

The nitrogen source in the fermentation medium according to the present disclosure is a nitrogen source conventionally used for *Duganella* sp., for example, one or more selected from the group consisting of potassium nitrate, sodium nitrate, ammonium nitrate, peptone, and yeast powder.

The exopolysaccharide according to the present disclosure, exopolysaccharide TDG3 produced by the *Duganella* sp. G3, consists of glucose, mannose, and galactose in a molar ratio of 3 : 2 : 1, with a glycosidic linkage of 4)-β-D-Glc*p*-(1→4)[α-D-Man*p*-(1→2)]-β-D-Man*p*-(1→4)-β-D-Glc*p*-(1→3)-β-D-Gal*p*-(1→4)-β-D-Glc*p*-(1→, and a structural formula of 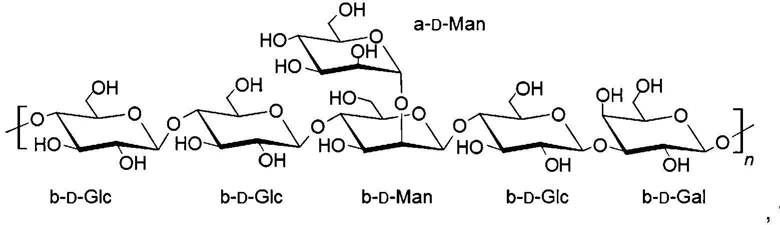 where n = 100 to 100000.

A method for producing the above exopolysaccharide TDG3 includes the following steps:
diluting the fermentation broth of *Duganella* sp. G3 by adding water, removing proteins, then adding 2 to 3 times the volume of ethanol to a supernatant, collecting a precipitate by centrifugation, and drying the precipitate to obtain pure exopolysaccharide TDG3.

The method for removing proteins according to the present disclosure is a method conventionally used in the art and includes, for example, one or more steps selected from the group consisting of adding 0.05% to 0.2% NaOH, filtering using a 20 nm to 2 µm filter membrane, and ultrafiltering using an ultrafiltration membrane with a molecular weight cut-off of 5 kD to 50 kD.

In specific embodiments of the present disclosure, the method for producing the above exopolysaccharide TDG3 includes the following specific steps: mixing the fermentation broth of *Duganella* sp. G3 with twice the volume of pure water, then adding NaOH to a final concentration of 0.1% to 0.2%, boiling a resulting mixture for 10 to 20 min, then filtering a precipitate through a 1 µm filter membrane, finally adding 0.1% to 0.3% sodium acetate and 3 times the volume of ethanol, performing uniform stirring, collecting the precipitate by centrifugation, and drying the precipitate to obtain the pure exopolysaccharide TDG3.

The present disclosure provides the use of the above exopolysaccharide TDG3 in the manufacture of a medicament for treating obesity.

The obesity according to the present disclosure is obesity generally known in the art, and the causative factors thereof include, but are not limited to, environmental factors, such as diet, lack of physical exercise, ultra-processed foods, fast food, microbiome, and chemical pollutants. The obesity phenotypes include, but are not limited to, subtypes such as metabolically healthy obesity, metabolically abnormal obesity, metabolic abnormalities, normal-weight obesity, and sarcopenic obesity.

The medicament for treating obesity according to the present disclosure may be administered in the form of intraperitoneal injection or subcutaneous injection.

The medicament for treating obesity according to the present disclosure may be administered to any animal with obesity. These animals include humans and non-human animals, such as pets or livestock.

The concentration and dose of administration of the medicament for treating obesity according to the present disclosure depend on the age, health and body weight of a recipient, the frequency of treatment, the route of administration, etc.

The pharmaceutical carrier used in the medicament for treating obesity according to the present disclosure may be a pharmaceutical carrier conventionally used in the art, such as an isotonic NaCl solution, an isotonic glucose solution, or an isotonic solution containing a buffer system, such as phosphate-buffered saline (PBS).

In specific embodiments of the present disclosure, the pharmaceutical carrier used is 0.9% physiological saline.

Compared with the prior art, embodiments of the present disclosure have the following advantages:
(1) the present disclosure has discovered for the first time a strain of *Duganella* sp. G3, and the exopolysaccharide TDG3 produced thereby, as a weight-loss drug, may effectively improve obesity phenotypes in mice, is safe and non-toxic, has strong efficacy, and shows good medicinal prospects;
(2) the exopolysaccharide TDG3 of the present disclosure may reduce body weight by suppressing appetite and reducing energy intake;
(3) the exopolysaccharide TDG3 of the present disclosure may alleviate excessive fat accumulation with less impact on muscle composition, and no adverse effects on the liver and cardiovascular system;
(4) the exopolysaccharide TDG3 of the present disclosure, as a TLR4/DC-SIGN dual receptor agonist, may bind to both TLR4 and DC-SIGN receptors, resulting in simultaneous activation of dual signaling pathways and significant body weight reduction.

### BRIEF DESCRIPTION OF THE DRAWINGS

FIG. 1 shows an infrared absorption spectrum of the exopolysaccharide TDG3 produced by *Duganella* sp. G3;
FIG. 2 shows a liquid chromatogram for component determination after derivatization of the exopolysaccharide TDG3, where the labels in the figure Man represents mannose, Glc represents glucose, Gal represents galactose;
FIG. 3 shows a gas chromatogram of the exopolysaccharide TDG3 after methylation and acetylation treatment;
FIGS. 4A-4B show the heteronuclear single-quantum coherence spectrum (FIG.4A) and the heteronuclear multiple bond correlation spectrum (FIG. 4B) of the exopolysaccharide TDG3;
FIG. 5 shows representative photographs of the body shapes of mice in each experimental group, where ND shows a photograph of the body shape of mouse in the normal diet group, HFD shows a photograph of the body shape of obese mouse fed a high-fat diet, and HFD+LG and HFD+HG show photographs of the body shapes of obese mice fed a high-fat diet after intraperitoneal injection of TDG3 at doses of 2 mg/kg and 5 mg/kg, respectively, once per week for three consecutive weeks;
FIG. 6 shows line charts of changes in body composition of mouse skin, where the left panel shows a line chart of changes in body weight, the middle panel shows a line chart of changes in fat mass, and the right panel shows a line chart of changes in lean mass;
FIG. 7 shows the total food take of mice in each experimental group over a 21-day experimental period;
FIG. 8 shows MST analysis of the interaction between exopolysaccharide TDG3 and DC-SIGN, and between exopolysaccharide TDG3 and TLR4;
FIG. 9 shows an *in vivo* safety evaluation of the exopolysaccharide TDG3.

### DETAILED DESCRIPTION OF THE EMBODIMENTS

Unless otherwise defined, all technical and scientific terms used in the present disclosure have the meanings commonly understood by those of ordinary skill in the art to which this disclosure belongs. The specific embodiments and examples described below are for illustrative purposes only and are not intended to limit the present disclosure. Unless otherwise specified, the reagents or materials used in the following examples can be purchased commercially or synthesized by referring to existing methods. The present disclosure is further described in detail below in conjunction with examples and the accompanying drawings.

Example 1 Screening of *Duganella* sp. G3, identification of 16S sequence, and fermentation and purification of exopolysaccharide
1. Screening of *Duganella* sp. G3:
   (1) The formulation of the screening medium was: KH₂PO₄ 1 g/L, CaCl₂ 0.1 g/L, MgCl₂·6H₂O 0.3 g/L, FeSO₄ 0.0125 g/L, KNO₃ 2 g/L, sucrose 20 g/L, pH 7.2; for the solid medium, agar 15 g/L was additionally added; and the medium was sterilized at 121°C for 20 min.
   (2) Screening method: soil sourced from a garden in Nanjing was placed in a 2 mL EP tube and 1 mL of physiological saline was added. The resulting mixture was shaken uniformly, and centrifuged at 50 g for 1 min. The precipitated soil particles were removed, and 500 µL of the supernatant was aspirated, and centrifuged at 12000 g for 3 min. The supernatant was discarded and the bacterial pellet were then pipetted and rinsed with 1 mL of physiological saline, centrifuged, and finally dissolved in 200 µL of physiological saline to prepare a soil leachate. The soil leachate was diluted and spread on a solid plate medium, and cultured at 28°C for 2 to 3 days. After the colonies appeared, a single colony producing viscous polysaccharide on the surface was picked based on the morphological characteristics of the colony. The colonies were diluted and spread on a fresh solid medium, and cultured at 28°C. After the colony appeared again, a single colony was picked, diluted, and spread, and this process was repeated until the colony growing on the solid medium exhibited a uniform morphology. The fermentation potential of the colony was tested using a liquid screening medium.
2. Molecular identification: the 16S rDNA (SEQ ID NO. 1 in the sequence listing) of the selected single colony strain was subjected to Blast alignment. According to the Blast alignment results, which showed a sequence similarity of up to 99.02% to *Duganella* sp. PAMC 2743, the single colony was identified as *Duganella* sp., designated as *Duganella* sp. G3.
3. Method for producing and purifying the exopolysaccharide TDG3 produced by *Duganella* sp. G3:
   (1) The formulation of the fermentation broth used was: sucrose 20 to 50g/L, KNO₃ 1 to 4 g/L, NaH₂PO₄ 0.5 to 2 g/L, CaCl₂ 0.02 to 0.1 g/L, MgSO₄·7H₂O 0.1 to 0.5 g/L, FeSO₄·7H₂O 0.01 to 0.06 g/L, MnSO₄·H₂O 0.005 to 0.01 g/L, ZnCl₂ 0.01 to 0.02 g/L, pH 7; and the solid medium was prepared by adding 15 g/L agar powder on the basis of liquid medium components.
   (2) Fermentation process: a single colony was first picked onto a solid plate, and cultured at a temperature of 28°C and a rotation speed of 230 rpm for 2 days to obtain a high-viability colony. The colony was then picked into 10 mL of liquid medium, and cultured at a temperature of 28°C and a rotation speed of 230 rpm for 2 days to serve as a seed culture; then the fermentation was scaled up at an inoculation ratio of 2%; and the culture was continued for 2 to 3 days to obtain a high-viscosity fermentation broth.
   (3) Purification: purification was performed using NaOH and filtration, with the specific process as follows. The obtained fermentation broth was mixed with twice the volume of pure water; NaOH was then added to a final concentration of 0.1%; the resulting mixture was boiled for 20 min; and then the precipitate was filtered through a 1 µm filter membrane. Finally, 0.2% sodium acetate and 3 times the volume of ethanol were added and uniform stirred; and the precipitate was collected by centrifugation, and dried to obtain the pure exopolysaccharide TDG3.

### Example 2 Structural analysis of exopolysaccharide TDG3

For the pure exopolysaccharide TDG3, the structure was analyzed and deduced using techniques including infrared spectroscopy, high-performance liquid chromatography, gas chromatography-mass spectrometry, and nuclear magnetic resonance. The structural analysis was as follows.

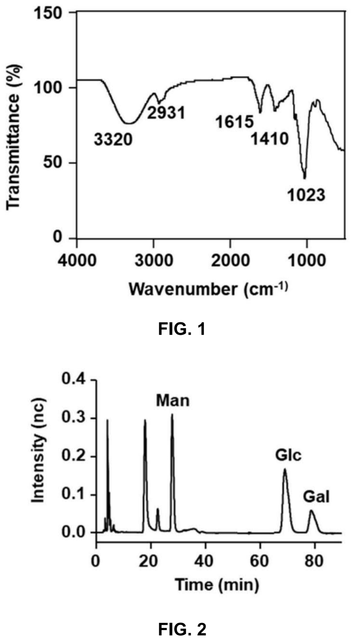

The pure exopolysaccharide TDG3 was tested using an infrared scanning instrument NICOLETIS^{™} 10 (Thermo Fisher Scientific) to analyze the existing functional groups thereof. The specific testing process was as follows: a pressed potassium bromide blank pellet was placed into the sample holder of the infrared scanner sample compartment, the reference background spectrum was confirmed to be acquired, then a test sample was placed into the spectrometer, and the sample was scanned, where the sample used each time was 2 to 5 mg. The infrared test results are shown in FIG. 1. The pure exopolysaccharide TDG3 has significant absorption peaks at 3320, 2931, 1615, 1410, and 1023 cm⁻¹, where the broad absorption peak at 3320 cm⁻¹ belongs to hydroxyl groups in various environments within the polysaccharide molecule; the absorption peak at 2931 cm⁻¹ comes from the stretching and bending vibrations of C-H in the saccharide ring; the absorption peak at 1615 cm⁻¹ comes from hydrogen bonds formed by crystalline water and saccharide; the absorption peak at 1410 cm⁻¹ comes from the O-C-O bond in the acetal group; the absorption peak at 1023 cm⁻¹ comes from the C-C bond in the pyranose ring.

High-performance liquid chromatography (HPLC) was used to quantitatively analyze the various monosaccharides present in the pure exopolysaccharide TDG3. The process was as follows: after complete acid hydrolysis of the exopolysaccharide TDG3 with trifluoroacetic acid, 1-Phenyl-3-methyl-5-pyrazolone (PMP) derivatization, chloroform extraction, and collection and filtration of aqueous phase, analysis was performed by HPLC using a Waters^{™} HPLC system (Waters Inc., USA) equipped with a Zorbax^{®} SB-Aq column (4.6 mm × 150 mm, Agilent Technologies, Inc., USA). FIG. 2 shows the liquid chromatogram for monosaccharide component determination. It can be seen from FIG. 2 that the exopolysaccharide TDG3 is composed of glucose, mannose, and galactose in a molar ratio of 3 : 2 : 1.

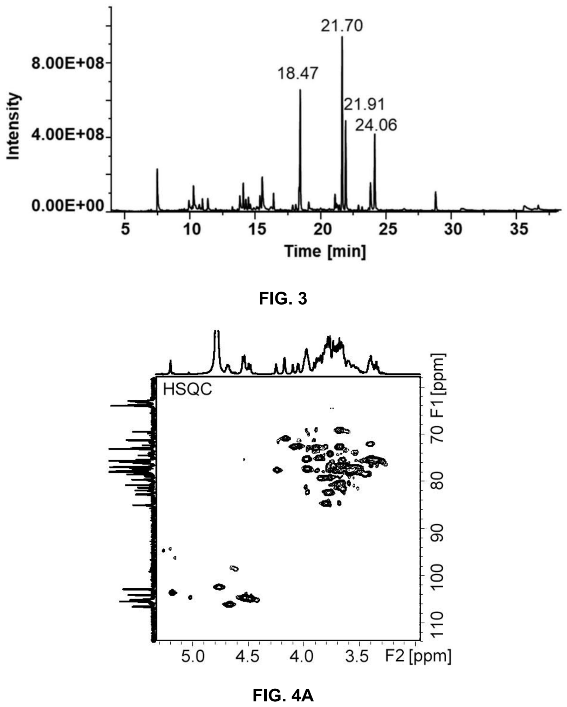

Gas chromatography-mass spectrometry was used to analyze the type of glycosidic linkage in the exopolysaccharide TDG3. The process was as follows: exposed hydroxyl groups in the polysaccharide were labeled by methylation reaction, then the polysaccharide was hydrolyzed and the glycosidic bonds were cleaved to expose the hydroxyl groups corresponding to the glycosidic bonds, and these hydroxyl groups were modified by acetylation. After the above steps, the polysaccharide was converted into a variety of volatile and thermally stable monosaccharide derivatives; and the monosaccharide derivatives differing in the number or spatial position of acetyl groups were separated using a gas chromatograph (Thermo Scientific ISQ LT, USA) equipped with a TG-200MS column to identify the type of glycosidic linkage in the polysaccharide. The gas chromatography results are shown in FIG. 3. Combined with the mass spectrometry data, it can be seen that the exopolysaccharide TDG3 is composed of T-Manp with a peak time of 18.5 min, 1,4-linked Glcp with a peak time of 21.7 min, 1,3-linked Manp with a peak time of 21.9 min, and 1,2,4-linked Manp with a peak time of 24.1 min.

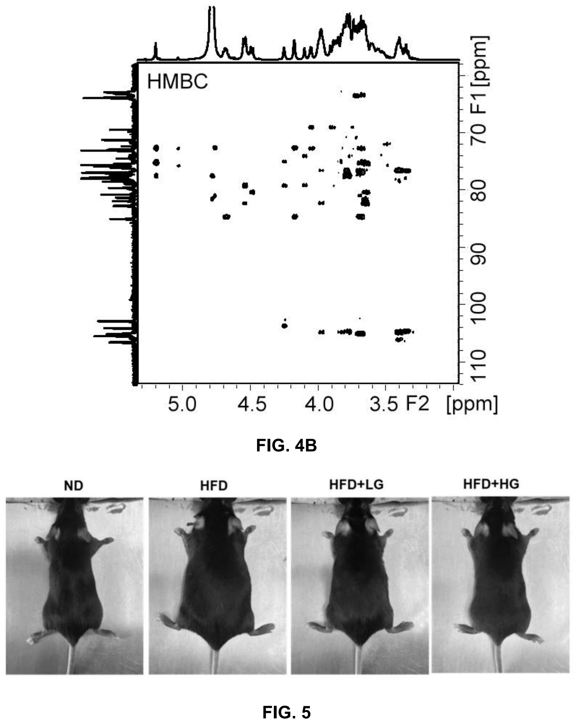

Nuclear magnetic resonance spectroscopy was used to analyze the linkage sequence of the exopolysaccharide TDG3. The process was as follows: after partial acid hydrolysis of the exopolysaccharide TDG3 with trifluoroacetic acid, D₂O was added for dissolving, and deuteration treatment was performed. Finally, trimethylsilylpropanoic acid (TMSP) was added as an internal standard; Nuclear magnetic resonance (NMR) analysis was performed using a Bruker^{®} Avance 500MHz analyzer (Bruker, Karlsruhe, Germany), with heteronuclear single-quantum coherence spectrum (HSQC) and heteronuclear multiple bond correlation spectrum (HMBC) as shown in FIGS. 4A-4B; and the ¹H NMR and ¹³C NMR shifts (ppm) of TDG3 were assigned, with the results shown in Table 1.

**Table 1 ¹H NMR and ¹³C NMR shifts (ppm) of TDG3**

| | Chemical shift (ppm) | | | | |
|---|---|---|---|---|---|
| | H1/C1 | H2/C2 | H3/C3 | H4/C4 | H5/C5 |
| α-D-Man(1→ | 5.19 | 4.05 | 3.90 | 3.68 | 3.97 |
| | 104.1 | 72.8 | 73.2 | 69.5 | 75.6 |
| → 2,4)β-D-Man(1→ | 4.76 | 4.24 | 3.86 | 3.77 | 3.55 |
| | 102.8 | 78.0 | 75.4 | 79.6 | 77.9 |
| → 4)β-D-Glc(1→ | 4.67 | 3.40 | 3.65 | 3.66 | 3.52 |
| | 106.5 | 75.7 | 77.0 | 82.0 | 77.5 |
| → 4)β-D-Glc(1→ | 4.53 | 3.35 | 3.64 | 3.68 | 3.97 |
| | 105.1 | 75.9 | 77.1 | 80.9 | 77.7 |
| → 4)β-D-Glc(1→ | 4.52 | 3.40 | 3.65 | 3.78 | 3.97 |
| | 105.3 | 75.8 | 77.0 | 82.7 | 77.7 |
| → 3)β-D-Gal(1→ | 4.49 | 3.68 | 3.80 | 4.17 | 3.70 |
| | 105.4 | 73.0 | 85.1 | 71.2 | 78.0 |

Example 3 *In vivo* dose-dependent weight loss effect in mice after one-week administration of exopolysaccharide TDG3

### (1) Establishment of animal model:

Experimental animals were male C57BL/6 mice, 6-8 weeks old, and weighing 20 ± 2 g. The animals were housed under standard experimental conditions with a 12-hour light-12-hour dark cycle and had *ad libitum* access to water and food. At the start of the study, the mice were randomly divided into 10 groups based on body weight. Group 1 was maintained on a normal diet (ND), while group 2 to group 10 were provided with a high-fat diet to induce a mouse obesity model. After 8 weeks, the mice in group 2 (HFD) were not injected with TDG3, and the mice in group 3 to group 10 were intraperitoneally injected with TDG3 once at doses of 0.5, 1, 2, 5, 10, 20, 50, and 100 mg/kg, respectively.

### (2) Tracking of mouse body weight

Data on mouse body weight changes were measured and statistically analyzed one week after the intraperitoneal injection of TDG3.

### (3) Result analysis

As shown in Table 2, after one week, the mouse body weight in both the CK group and the HFD group increased significantly; however, in obese mice intraperitoneally injected with TDG3 at a dose of 0.5 mg/kg, the magnitude of body weight increase after one week was significantly reduced compared with that of the mice in HFD group; when the TDG3 injection dose reached 1 mg/kg, the mouse body weight began to decrease; furthermore, within the dose range of 1 mg/kg, 2 mg/kg, and 5 mg/kg, the magnitude of mouse body weight decrease exhibited a dose-dependent manner; and when the TDG3 injection dose was further increased from 5 mg/kg, the magnitude of mouse body weight decrease exhibited no significant change compared with that at the dose of 5 mg/kg.

**Table 2 Statistics of mouse body weight change relative to initial body weight after one week**

| Group | Body weight change (g) |
|---|---|
| CK | 0.37 ± 0.27 |
| HFD | 1.86 ± 0.33 |
| 0.5 mg/kg | 0.46 ± 0.39 |
| 1 mg/kg | -1.02 ± 0.28 |
| 2 mg/kg | -2.30 ± 0.30 |
| 5 mg/kg | -3.49 ± 0.49 |
| 10 mg/kg | -3.71 ± 0.55 |
| 20 mg/kg | -3.76 ± 0.43 |
| 50 mg/kg | -3.68 ± 0.52 |
| 100 mg/kg | -3.82 ± 0.61 |

Example 4 Weight losing effect of exopolysaccharide TDG3 on obese mice after three-week consecutive administration

### (1) Establishment of animal model:

Experimental animals were male C57BL/6 mice, 6-8 weeks old, and weighing 20 ± 2 g. The animals were housed under standard experimental conditions with a 12-hour light-12-hour dark cycle and had *ad libitum* access to water and food. At the start of the study, the mice were randomly divided into four groups based on body weight. Group 1 was maintained on a normal diet (ND), while groups 2, 3, and 4 were provided with a high-fat diet (HFD). After 8 weeks, the mice in group 3 and group 4 were intraperitoneally injected with TDG3 at dose of 2 mg/kg (HFD+LG) and 5 mg/kg (HFD+HG), respectively, once per week for three consecutive weeks.

### (2) Tracking of mouse body weight and fat mass

During the 21-day experiment, the body weight of each mouse was measured daily, and the fat mass and lean mass of mice were quantified using a desktop NMR instrument.

### (3) Tracking of mouse food take

During the 21-day experiment, the food take of each mouse was measured daily, and the total food take of each mouse was calculated.

### (4) Data analysis

Data processing was performed using the software Graphpad Prism 6.0, and statistical analysis for comparisons among multiple groups was performed using the One-way ANOVA statistical method. P < 0.05 was considered statistically significant.

### (5) Result analysis

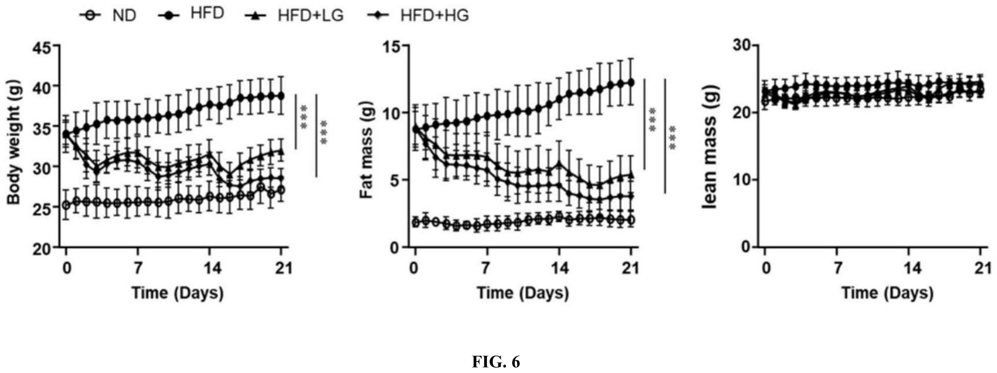

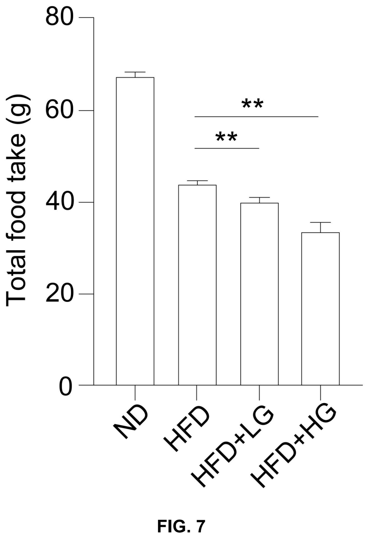

As shown in FIG. 5 and FIG. 6, intraperitoneal injection of TDG3 significantly alleviated the obesity phenotypes in mice induced by high-fat feeding and had less effect on the lean mass of mice. Furthermore, it can be seen from FIG. 7 that the food take of mice during the 21-day experiment was significantly reduced, demonstrating that TDG3 achieved the purpose of reducing obesity in mice by reducing energy intake.

Example 5 Establishment of TDG3 as TLR4/DC-SIGN dual receptor glycoligand

### (1) Establishment of cell model

pmCherry-N1 plasmid encoding TLR4 or DC-SIGN was transfected into NIH-3T3 cells using Lipofectamine^{™} 2000 transfection kit (Invitrogen). After 48 hours of incubation, the transfected cells were harvested.

### (2) Microscale thermophoresis (MST) analysis

After cell membrane protein extraction, microscale thermophoresis experiments were performed using a Monolith NT.115 system at 20% excitation power and 90% MST power.

### (3) Result analysis

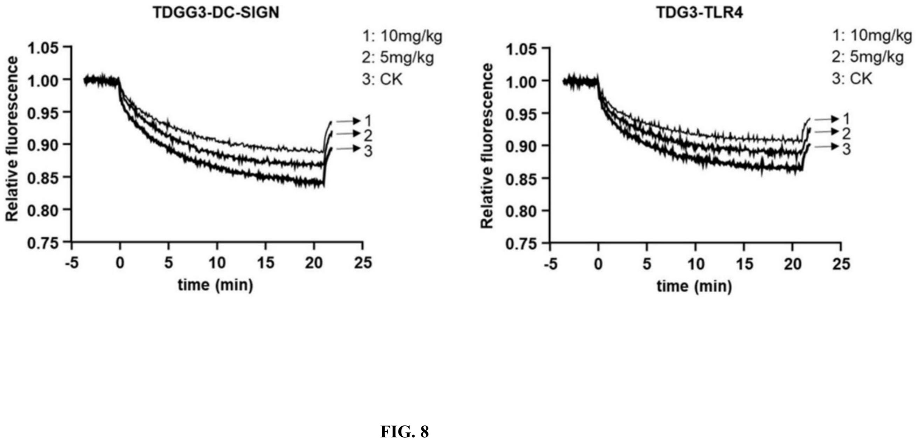

The MST analysis results of the interaction between TDG3 and TLR4, and between TDG3 and DC-SIGN are shown in FIG. 8. TDG3 exhibits high affinity for both TLR4 and DC-SIGN, indicating that TDG3 is a potential glycoligand for the TLR4/DC-SIGN dual receptors.

Example 6 Safety analysis of exopolysaccharide TDG3

### (1) Establishment of animal model

Mice were intraperitoneally administered TDG3 (200 mg/kg) once per week for three consecutive weeks. Subsequently, the safety of mice was assessed by measuring key biomarkers related to liver function and cardiovascular health in serum biochemical indicators. Data processing was performed using the software Graphpad Prism^{™} 6.0, and statistical analysis for comparisons among multiple groups was performed using the One-way Analysis of Variance (ANOVA) statistical method. P < 0.05 was considered statistically significant.

### (2) Result analysis

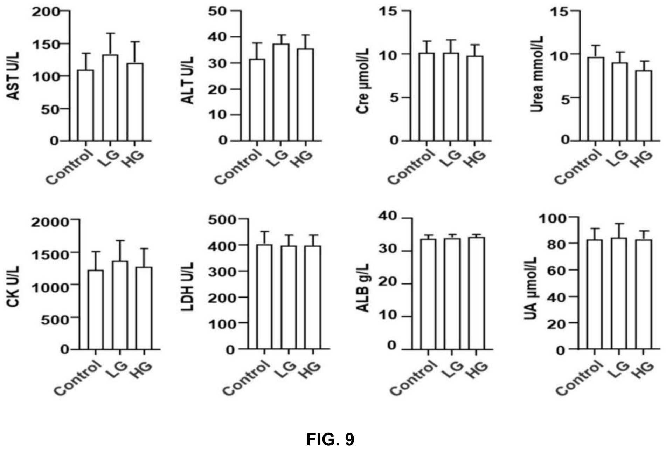

Biochemical indicators related to liver function and cardiovascular health were assessed, including aspartate aminotransferase (AST), alanine aminotransferase (ALT), creatinine (Cre), urea (Urea), creatine kinase (CK), lactate dehydrogenase (LDH), albumin (ALB), and uric acid (UA). No significant changes were observed in these biomarkers (FIG. 9), demonstrating that TDG3 caused no adverse effects on liver function and cardiovascular function.

## Claims

1. A strain of *Duganella* sp., which is a strain of *Duganella* sp. G3, with a deposit number of China Center for Type Culture Collection (CCTCC) NO: M 2024137.

2. A method for culturing the *Duganella* sp. of claim 1, wherein the method specifically comprises: inoculating the *Duganella* sp. G3 into a fermentation medium for culture, wherein the culture is performed at a pH of 5 to 9, and a temperature of 26°C to 32°C, and a formulation of the fermentation medium comprises carbon source 20 to 50 g/L, nitrogen source 1 to 4 g/L, NaH₂PO₄ 0.5 to 2 g/L, CaCl₂ 0.02 to 0.1 g/L, MgSO₄·7H₂O 0.1 to 0.5 g/L, FeSO₄·7H₂O 0.01 to 0.06 g/L, MnSO₄·H₂O 0.005 to 0.01 g/L, and ZnCl₂ 0.01 to 0.02 g/L.

3. The culture method of claim 2, wherein the carbon source is one or more selected from the group consisting of glucose, sucrose, and starch, and the nitrogen source is one or more selected from the group consisting of potassium nitrate, sodium nitrate, ammonium nitrate, peptone, and yeast powder.

4. An exopolysaccharide TDG3, wherein the exopolysaccharide TDG3 is composed of glucose, mannose, and galactose in a molar ratio of 3 : 2 : 1, with a glycosidic linkage of 4)-β-D-Glc*p*-(1→4)[α-D-Man*p*-(1→2)]-β-D-Man*p-*(1→4)-β-D-Glc*p*-(1→3)-β-D-Gal*p*-(1→4)-β-D-Glc*p*-(1→, and a structural formula of 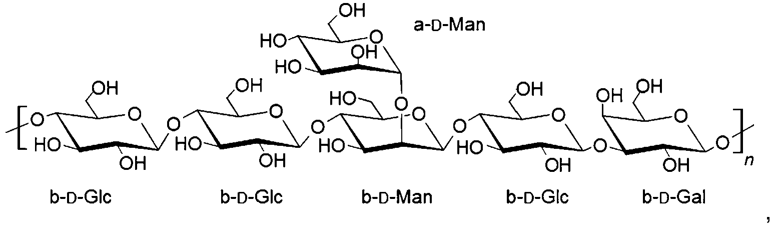 wherein n = 100 to 100000.

5. A method for producing the exopolysaccharide TDG3 of claim 4, comprising the following steps:
diluting a fermentation broth of *Duganella* sp. G3 by adding water, removing proteins, then adding 2 to 3 times the volume of ethanol to a supernatant, collecting a precipitate by centrifugation, and drying the precipitate to obtain a pure exopolysaccharide TDG3.

6. The method of claim 5, wherein a process for removing proteins comprises one or more steps selected from the group consisting of adding 0.05% to 0.2% NaOH, filtering using a 20 nm to 2 µm filter membrane, and ultrafiltering using an ultrafiltration membrane with a molecular weight cut-off of 5 kD to 50 kD.

7. The method of claim 6, wherein the process specifically comprises: mixing the fermentation broth of *Duganella* sp. G3 with twice the volume of pure water, then adding NaOH to a final concentration of 0.1% to 0.2%, boiling a resulting mixture for 10 to 20 min, then filtering a precipitate through a 1 µm filter membrane, finally adding 0.1% to 0.3% sodium acetate and 3 times the volume of ethanol, performing uniform stirring, collecting the precipitate by centrifugation, and drying the precipitate to obtain the pure exopolysaccharide TDG3.

8. The exopolysaccharide TDG3 of claim 4 for use in treating obesity.

9. The exopolysaccharide TDG3 for use of claim 8, wherein the exopolysaccharide TDG3 is administered as a medicament in the form of intraperitoneal injection or subcutaneous injection; and the medicament is administered to a subject who is a human or a non-human animal.

10. The exopolysaccharide TDG3 for use of claim 8, wherein a pharmaceutical carrier used in the medicament is an isotonic NaCl solution, an isotonic glucose solution, or an isotonic solution containing a buffer system.
